# EUROPEAN PATENT APPLICATION

(11) **EP 1 721 975 A1**
(43) Date of publication of application: **15.11.2006**
(21) Application number: 05719187.6
(22) Date of filing: 16.02.2005
(51) Int. Cl.: C12N 15/11, C12Q 1/68

(54) **METHOD OF DETECTING AND IDENTIFYING GRAM-NEGATIVE OBLIGATIVE ANAEROBIC BACTERIUM**

(30) Priority: 17.02.2004 JP 2004040376
(71) Applicant: Sapporo Breweries Limited, Tokyo 150-8522 (JP)
(72) Inventor: NAKAKITA, Yasukazu, c/o Sapporo Breweries Limited, Yaizu-shi, Shizuoka 4250013 (JP); TSUCHIYA, Youichi, c/o Sapporo Breweries Limited, Yaizu-shi, Shizuoka 4250013 (JP)
(74) Representative: Vossius & Partner
(86) International application number: PCT/JP2005/002335
(87) International publication number: WO 2005/078092

(57) **Abstract**

There is provided a method of detecting potentially beer-spoiling obligatory anaerobic gram-negative bacteria, and a method of simultaneously detecting and distinguishing different beer-spoilage microorganisms including such bacteria.

## Description

### Technical Field

The present invention relates to a method for detecting and distinguishing obligatory anaerobic gram-negative bacteria.

### Background Art

As a greater proportion of beer produced in recent years is draft beer, a new awareness has risen regarding the freshness of beer. Under such circumstances, beer brewing companies are being forced to rapidly and accurately identify microbial contaminants that spoil beer (beer-spoilage microorganisms) in order to dramatically shorten the time from beer production to shipping.

Obligatory anaerobic bacteria belonging to *Pectinatus* spp. and *Megasphaera* spp. are responsible for higher risk of bacterial contamination with increased anaerobic conditions of beer products, and rapid and accurate detection of these bacteria is desirable. Methods known in the prior art for detecting such these bacteria include detection methods utilizing antigen-antibody reaction (for example, see Non-patent document 1), detection methods using PCR (Polymerase Chain Reaction) (for example, see Patent document 1) and detection methods using FISH (Fluorescent *in situ* Hybridization) (for example, see Patent document 2).

[Patent document 1] Japanese Patent Re-publication No. 09-820071
[Patent document 2] Japanese Unexamined Patent Publication No. 2001-145492
[Non-patent document 1] J. Am. Soc. Brew. Chem.: 51(4), 158-163, 1993

### Disclosure of the Invention

### Problems to be Solved by the Invention

However, the present inventors have discovered new beer-spoilage obligatory anaerobic gram-negative bacteria which may not be detectable by the aforementioned prior art methods.

It is therefore an object of the present invention to provide a method for detecting potentially beer-spoiling obligatory anaerobic gram-negative bacteria, which have not been detectable by prior art methods. It is another object of the present invention to provide a method for simultaneously detecting and distinguishing different types of beer-spoilage microorganisms including said bacteria.

### Means for Solving the Problems

In order to achieve the aforestated objects, the present invention provides a polynucleotide comprising all or a portion of the nucleotide sequence as set forth in SEQ ID NO: 1.

The present invention further provides a primer set for detection of *Malephilus cerevisiae* consisting of an oligonucleotide comprising the nucleotide sequence as set forth in SEQ ID NO: 2 and an oligonucleotide comprising the nucleotide sequence as set forth in SEQ ID NO: 3, as well as a method for detecting *Malephilus cerevisiae* by gene amplification characterized by comprising a step of amplifying a nucleic acid fragment using the aforementioned primer set and a step of detecting the obtained nucleic acid fragment.

The present invention further provides a primer set for detecting and distinguishing beer-spoilage microorganisms consisting of an oligonucleotide comprising the nucleotide sequence as set forth in SEQ ID NO: 8, an oligonucleotide comprising the nucleotide sequence as set forth in SEQ ID NO: 3 and an oligonucleotide comprising the nucleotide sequence as set forth in SEQ ID NO: 6, a probe set for detecting and distinguishing beer-spoilage microorganisms consisting of an oligonucleotide comprising the nucleotide sequence as set forth in SEQ ID NO: 4, an oligonucleotide comprising the nucleotide sequence as set forth in SEQ ID NO: 7 and an oligonucleotide comprising the nucleotide sequence as set forth in SEQ ID NO: 5, a kit for detecting and distinguishing beer-spoilage microorganisms characterized by containing the aforementioned primer set and probe set, and a method for detecting and distinguishing beer-spoilage microorganisms characterized by comprising a step of amplifying a nucleic acid fragment using the aforementioned primer set and a step of measuring the melting temperature for hybrids of the obtained nucleic acid fragment and the probe set according to claim 4.

The present inventors have succeeded in isolating the potentially beer-spoiling obligatory anaerobic gram-negative bacterium *Malephilus cerevisiae,* which cannot be detected by known methods, and have shown that the nucleotide sequence of the 16S ribosomal RNA gene (16S rRNA gene) of *Malephilus cerevisiae* SBC8034 (unapproved name) is the nucleotide sequence as set forth in SEQ ID NO: 1. This strain has been deposited at the National Institute of Advanced Industrial Science and Technology, International Patent Organism Depositary (IPOD) (Tsukuba Central 6, 1-1, Higashi 1-chome, Tsukuba-shi, Ibaraki-ken, Japan 305-8566) on October 21, 2003 as FERM BP-08528.

By using a primer set for detection of *Malephilus cerevisiae,* it is possible to specifically amplify the 16S rRNA gene of *Malephilus cerevisiae,* thereby allowing detection *of Malephilus cerevisiae.*

By using the primer set for detecting and distinguishing beer-spoilage microorganisms it is also possible to amplify the 16S rRNA genes of different beer-spoilage microorganisms. Moreover, by forming hybrids between the obtained nucleic acid fragment and the probe set for detecting and distinguishing beer-spoilage microorganisms and measuring the melting points of the hybrids, it is possible to detect and distinguish beer-spoilage microorganisms based on differences in melting point since the melting point will differ depending on the type of spoilage microorganism from which the nucleic acid fragment is derived.

### Effect of the Invention

There are provided a method for detecting potentially beer-spoiling obligatory anaerobic gram-negative bacteria that have not been detectable by prior art methods, and a method for simultaneously detecting and distinguishing different beer-spoilage microorganisms including said bacteria.

### Brief Explanation of the Drawings

Fig. 1 shows melting curves for (a) *Megasphaera cerevisiae* JCM6129 and (b) *M cerevisiae* SBC8034 at 640 nm.
Fig. 2 shows melting curves for (c) *P. cerevisiiphilus* VTT-E-79105 and (d) *Pectinatus frisingensis* VTT-E-79100 at 710 nm.

### Best Mode for Carrying Out the Invention

Preferred embodiments of the present invention will now be described in detail.

### <Polynucleotides>

The polynucleotides of the present invention will now be explained. A polynucleotide according to the present invention is characterized by comprising all or a portion of the nucleotide sequence as set forth in SEQ ID NO: 1, but also included are polynucleotides comprising all or a portion of the sequence complementary to the nucleotide sequence as set forth in SEQ ID NO: 1. SEQ ID NO: 1 represents the nucleotide sequence of the 16S rRNA gene of *Malephilus cerevisiae,* which is a potentially beer-spoiling obligatory anaerobic gram-negative bacterium. The polynucleotides of the present invention are useful for detection of *Malephilus cerevisiae* and can be used as primers for detection of *Malephilus cerevisiae,* as primer-amplified nucleic acid fragments or as detection probes, as described hereunder. The polynucleotides of the present invention may also be chemically modified with fluorescent substances or the like.

When a polynucleotide of the present invention is used as a detection primer or detection probe for *Malephilus cerevisiae,* it is preferably an oligonucleotide with a nucleotide length of 15-25. Primers may be designed easily by a person skilled in the art, if necessary utilizing primer design assistant software.

According to the present invention, the terms "polynucleotide" and "oligonucleotide" encompass DNA, RNA and PNA (peptide nucleic acid). Polynucleotides and oligonucleotides of the invention may be synthesized by publicly known methods such as, for example, the phosphoramidite method.

### <Detection of Maleplzilus cerevisiae>

A primer set for detection of *Malephilus cerevisiae* and a detection method for *Malephilus cerevisiae* using the primer according to the present invention will now be explained. The primer set for detection of *Malephilus cerevisiae* according to the present invention is characterized by consisting of an oligonucleotide comprising the nucleotide sequence as set forth in SEQ ID NO: 2 and an oligonucleotide comprising the nucleotide sequence as set forth in SEQ ID NO: 3, and since both oligonucleotides have nucleotide sequences specific to the 16S rRNA gene of *Malephilus cerevisiae,* it is possible to specifically amplify the 16S rRNA gene of *Malephilus cerevisiae,* thereby permitting specific detection *of Malephilus cerevisiae.*

For detection of *Malephilus cerevisiae* by the detection method of the present invention, first nucleic acid is extracted from a sample (for example, a malt beverage such as beer or low-malt beer (happoshu)). The nucleic acid extraction may be carried out using a method known in the field, and specifically DNA may be extracted by, for example, a method involving phenol extraction and ethanol precipitation or a method employing glass beads, while RNA may be extracted by an AGPC method or guanidine/cesium chloride ultracentrifugation.

The obtained nucleic acid is then used as template for amplification of a nucleic acid fragment using the aforementioned primer set. The amplification method used may be an amplification method which is known in the field, and particularly PCR or RT-PCR is preferred. In the case of PCR, a nucleic acid fragment consisting of the nucleotide sequence of the portion of the 16S rRNA gene between the primer set is amplified by using DNA polymerase and the extracted DNA as template. In the PCR, a cycle consisting of denaturation, annealing and complementary chain synthesis is repeated to yield a nucleic acid fragment (double-stranded DNA), and the optimum PCR conditions such as the temperature and time for each step and the number of cycles may be easily determined by a person skilled in the art. In RT-PCR, extracted RNA is used as template for synthesis of cDNA with reverse transcriptase, and the obtained cDNA is then used as template for PCR.

The amplified nucleic acid fragment is detected next. Specifically, it is judged whether or not the amplified nucleic acid fragment is specific to *Malephilus cerevisiae.* The detection may be carried out by a method known in the field, and for example, it may be carried out by hybridization using a probe that specifically hybridizes to *Malephilus cerevisiae.* Alternatively, the nucleic acid fragment may be detected by measuring the melting temperature of the amplified nucleic acid fragment.

The measurement of melting temperature may be performed by a method known in the field, and for example, the melting temperature may be measured by adding a nucleic acid staining reagent such as SYBR Green I to a solution containing the amplified nucleic acid fragment, measuring the fluorescent intensity continuously while raising the temperature of the solution, and analyzing the obtained melting temperature curve to determine the melting temperature. Since the nucleic acid staining reagent may be mixed with the reaction solution used for amplification of the nucleic acid fragment, it is possible to detect the nucleic acid fragment immediately after the nucleic acid fragment amplification reaction has been completed. Thus, since it allows the step of amplifying the nucleic acid fragment and the step of detecting the obtained nucleic acid fragment to be carried out continuously in the same tube or capillary, it is particularly preferred to use a method of measuring the melting temperature of the amplified nucleic acid fragment using a nucleic acid staining reagent. This method also has the advantage of high detection sensitivity.

The melting temperature of the nucleic acid fragment derived from *Malephilus cerevisiae* which is amplified from the primer set for detection of *Malephilus cerevisiae* according to the present invention is approximately 88°C, and comparison with the melting temperature of the sample will permit judgment of whether or not *Malephilus cerevisiae* is contained in the sample, thereby allowing detection of *Malephilus cerevisiae.*

### <Detecting and distinguishing of beer-spoilage microorganisms>

A primer set, probe set and kit for detecting and distinguishing beer-spoilage microorganisms and a method for detecting and distinguishing beer-spoilage microorganisms using them will now be explained.

The primer set for detecting and distinguishing beer-spoilage microorganisms according to the present invention is characterized by comprising oligonucleotides consisting of the nucleotide sequences as set forth in SEQ ID NOS: 8, 3 and 6, respectively. The oligonucleotide consisting of the nucleotide sequence as set forth in SEQ ID NO: 8 is a universal primer for the 16S rRNA gene. By using the primer set of the present invention it is possible to amplify nucleic acid fragments of different beer-spoilage microorganisms.

The probe set for detecting and distinguishing beer-spoilage microorganisms according to the present invention is characterized by comprising oligonucleotides consisting of the nucleotide sequences as set forth in SEQ ID NOS: 4, 7 and 5, respectively. As described below, the probe set can detect nucleic acid fragments of different beer-spoilage microorganisms, so that beer-spoilage microorganisms can be detected and distinguished.

The kit for detecting and distinguishing beer-spoilage microorganisms according to the present invention is characterized by comprising the aforementioned primer set and probe set. The kit may also contain a reaction buffer, dNTP mixture and enzymes, as well as a DNA extraction reagent.

In order to detect and distinguish beer-spoilage microorganisms by the detecting and distinguishing method of the present invention, first nucleic acid is extracted from a sample (for example, a malt beverage such as beer or low-malt beer (happoshu)). The nucleic acid extraction may be carried out in the same manner as described above.

The obtained nucleic acid is then used as template for amplification of a nucleic acid fragment using the aforementioned primer set. The amplification may be carried out by the same method as described above, and PCR or RT-PCR is preferred.

Next, the obtained nucleic acid fragment and the probe set are hybridized and the hybrid melting temperature is measured. The principle for measurement of the melting temperature is explained below. The oligonucleotides consisting of the nucleotide sequences as set forth in SEQ ID NOS: 4 and 7 are labeled at their respective 5' ends with the fluorescent substances LC Red640 and LC Red705 (hereinafter these will be referred to as "Red640 probe" and "Red705 probe", respectively). While the oligonucleotide consisting of the nucleotide sequence as set forth in SEQ ID NO: 5 is labeled at the 3' end with FITC (hereinafter, this will be referred to as "FITC probe"). Each probe is designed to hybridize with the nucleic acid fragment of the beer-spoilage microorganism in a manner with the 3' end of the FITC probe and the 5' ends of the Red640 probe and Red705 probe in proximity. When light having the excitation wavelength for FITC is irradiated on the hybrid with both the FITC probe and the Red640 probe (or Red705 probe) hybridized to the nucleic acid fragment, FRET (Fluorescence Resonance Energy Transfer) occurs and light of the fluorescent wavelength of Red640 (or Red705) is observed. When the temperature is raised in this state, the FITC probe and/or Red640 probe (or Red705 probe) melts and separates from the nucleic acid fragment, resulting in cessation of FRET and reduction in the fluorescent intensity of Red640 (or Red705). The fluorescent intensity is measured at each temperature, and by plotting temperature on the horizontal axis and fluorescent intensity (including changing ratio) on the vertical axis, a melting curve is obtained. Analysis of the melting curve obtained in this manner permits the hybrid melting temperature to be determined.

The FITC probe and/or Red640 probe (or Red705 probe) are set so that the degree of mismatch with the nucleic acid fragment differs depending on the type of beer-spoilage microorganism. Thus, since the melting curve and melting temperature exhibited by the hybrid will differ depending on the type of beer-spoilage microorganism, the type of beer-spoilage microorganism can be distinguished based on the difference. Specifically, the melting temperatures exhibited are approximately 65°C for *Malephilus cerevisiae,* approximately 48°C and approximately 56°C for *Megasphaera cerevisiae,* approximately 63°C for *Pectinatus frisingensis* and approximately 54°C for *Pectinatus cerevisiiphilus.* Comparing these melting temperatures with the melting temperature of the sample will allow the beer-spoilage microorganism in the sample to be detected and distinguished.

Since the probe set of the present invention can be mixed with the reaction solution used for amplification of the nucleic acid fragment, the melting temperature can be measured immediately after completion of the nucleic acid fragment amplification reaction. Thus, the detecting and distinguishing method for beer-spoilage microorganisms according to the present invention has the advantage of allowing the step of amplifying the nucleic acid fragment and the step of measuring the melting temperature to be carried out continuously in the same tube or capillary.

### Examples

The present invention will now be explained in greater detail using examples, with the understanding that the invention is not limited to the examples.

(Example 1) Spoilage of beer by inoculation of *M. cerevisiae M. cerevisiae* SBC8034 was grown on GAM agar medium (Nissui Pharmaceutical Co., Ltd.) containing 0.2% malic acid. One loopful of *M cerevisiae* SBC8034 was inoculated into bottled all malt beer (pH 4.5, bitterness value: 30, alcohol content: 5%, volume: 350 ml), and the beer bottle was capped and culturing was carried out for about 1 month at 30°C, resulting in spoilage of the beer.

Table 1 shows the concentration (ppm) of organic acids in the spoiled beer after one month of culturing. The spoiled beer into which the *M cerevisiae* SBC8034 had been inoculated had approximately twice the concentration of malic acid or succinic acid compared to ordinary beer.

**[Table 1]**

| Organic acid | Normal beer | Spoiled beer | % (Spoiled/Normal) |
|---|---|---|---|
| Malic acid | 89 | 162 | 182 |
| Succinic acid | 66 | 168 | 255 |
| Lactic acid | 191 | 220 | 115 |
| Acetic acid | 158 | 221 | 140 |
| Propionic acid | <1 | <1 | - |

### (Example 2) 16S rRNA gene sequence

### (Preparation of genomic DNA)

*M cerevisiae* SBC8034 was inoculated into GAM agar medium containing 0.2% malic acid, and anaerobic culturing was conducted at 30°C for 7-14 days. The anaerobic culturing was carried out using an anaerobic culturing apparatus by Tabai Espec Corp., under conditions of N₂:H₂:CO₂ = 90:5:5. DNA was extracted from the anaerobic cultured *M. cerevisiae* SBC8034 cells using DNA extract reagent PrepMan Ultra (Applied Biosystems Japan, Ltd.).

### (Amplification and analysis of 16S rRNA gene)

The DNA extract prepared by the aforementioned method was used for sequencing of the 16S rRNA gene of *M. cerevisiae* SBC8034 using a MicroSeq Full Gene 16S rDNA kit (Applied Biosystems Japan, Ltd.). The sequence for the 16S rRNA gene of *M. cerevisiae* SBC8034 obtained as a result of the sequencing is as set forth in SEQ ID NO: 1. This gene sequence clearly differed from the gene sequences of the beer-spoiling obligatory anaerobic gram-negative bacteria *Pectinatus* spp. and *Megasphaera* spp.. A GenBank database search was also conducted, but no match was found with any registered gene sequence. It was thus demonstrated that *M cerevisiae* is a new beer-spoiling obligatory anaerobic gram-negative bacterium.

### (Example 3) Detection and distinction of M cerevisiae using real-time PCR

DNA was extracted from different potentially beer-spoiling bacterial strains by the same method as in Example 2, and the obtained DNA extracts were analyzed by PCR using oligonucleotides consisting of the nucleotide sequences as set forth in SEQ ID NO: 2 and 3 as primers, with the reaction reagent composition listed in Table 2.

**[Table 2]**

| Reagent | Volume |
|---|---|
| LightCycler-FastStart DNA Master SYBR Green 1* | 2.0 µL |
| Primer (10 µM) | (each) 1.0 µL |
| MgCl₂ (25 mM) | 1.6 µL |
| Sterilized water | 13.9 µL |
| DNA extract | 0.5 µL |
| Total | 20.0 µL |

| | |
|---|---|
| *: Roche Diagnostics K.K. | |

The PCR was conducted by using a LightCycler Quick System 330 (Roche Diagnostics, KK.) as the reaction apparatus by means of 10 minutes of treatment at 95°C followed by repetition of 40 cycles with each cycle consisting of 15 seconds at 95°C, 5 seconds at 50°C and 20 seconds at 72°C.

Upon completion of the PCR the temperature was raised to 95°C, and after immediate cooling to 65°C, the temperature was held for 15 seconds and the temperature was raised again to 95°C at a rate of 20°C/sec. During heating, the fluorescent intensity at 530 nm was measured at every 0.2°C, the negative of the first derivative of the value (-dF/dT) was plotted, and the resulting peak was used to determine the melting temperature.

Table 3 shows the presence or absence of peaks for a melting temperature of about 88°C for PCR products of different strains. As seen in Table 3, a peak for an approximately 88°C melting temperature was observed only for *M. cerevisiae.* This demonstrated that M *cerevisiae* can be specifically detected by using a primer set comprising oligonucleotides consisting of the nucleotide sequences as set forth in SEQ ID NO: 2 and 3.

Some of the strains used (isolated by Sapporo Breweries LTd.) were identified based on their 16S rRNA gene sequences by the same method as in Example 2.

**[Table 3]**

| Tested strains (Gram-negative bacteria) | 88°C peak | Tested strains (Gram-negative bacteria) | 88°C peak |
|---|---|---|---|
| Malephilus cerevisiae SBC8034 | + | Lactobacillus brevis SBC8003 | - |
| Malephilus cerevisiae SBC8065 | + | Lactobacillus brevis ID197-1 | - |
| Malephilus cerevisiae SBC8066 | + | Lactobacillus collinoides JCM1123 | - |
| Pectinatus cerevisiiphiuls VTT-E-79105 | - | Lactobacillus buchneri JCM1115 | - |
| Pectinatus frisingensis VTT-E-79100 | - | Lactobacillus lindneri VTT-E-82166 | - |
| Pectinatus frisingensis ID107-9 | - | Lactobacillus paracasei ID 196-1 | - |
| Megasphaera cerevisiae JCM6129 | - | Lactobacillus malefermentans ID 140-3 | - |
| Prevotella corporis JCM8529 - | | Lactobacillus plantarum ID158-1 | - |
| Zymomonas mobilis subsp. mobilis IF013756 | - | Lactobacillus coryniformis subsp. coryniformis JCM1164 | - |
| Enterobacter aerogenes JCM1235 | - | Pediococcus damnosus BC8022 | - |
| Klebsiella aerogenes ATCC15050 | - | Lactococcus lactis ID169-1 | - |
| Rahnella aquatilis ID252-2 | - | Staphylococcus warneri ID249 | - |
| Cedecea davisae ID313-9 | - | | |

| | | | |
|---|---|---|---|
| JCM: Japan Collection of Microorganisms, Saitama, Japan ATCC: American Type Culture Collection, USA VTT: Valtion Teknillinen Tutkimuskeskus, Finland SBC, ID: Isolated by Sapporo Breweries Ltd. | | | |

### (Example 4) Detection and distinction of obligatory anaerobic gram-negative bacteria using real-time PCR

DNA extracts from different strains prepared by the same method as in Example 2 were subjected to PCR with the reaction reagent composition listed in Table 4, using as primers an oligonucleotide consisting of the nucleotide sequence as set forth in SEQ ID NO: 8 (universal primer for 16S rRNA gene, 5'-TGGAGAGTTTGATCCTGGCTC-3') and oligonucleotides consisting of the nucleotide sequences as set forth in SEQ ID NO: 3 and 6, and using as probes an oligonucleotide consisting of the nucleotide sequence as set forth in SEQ ID NO: 4 (phosphorylated at the 3'-end and labeled with LC Red640 at the 5'-end), an oligonucleotide consisting of the nucleotide sequence as set forth in SEQ ID NO: 7 (phosphorylated at the 3'-end and labeled with LC Red705 at the 5'-end) and an oligonucleotide consisting of the nucleotide sequence as set forth in SEQ ID NO: 5 (labeled with FITC at the 3'-end).

**[Table 4]**

| Reagent | Volume |
|---|---|
| LightCycler-FastStart DNA Master Hybridization Probes* | 2.0 µL |
| Primer (10 µM) | (each) 1.0 µL |
| Probe (10 µM) | (each) 0.4 µL |
| MgCl₂ (25 mM) | 1.6 µL |
| Sterilized water | 11.7 µL |
| DNA extract | 0.5 µL |
| Total | 20.0 µL |

| | |
|---|---|
| *: Roche Diagnostics K.K. | |

The PCR was conducted by using a LightCycler Quick System 330 (Roche Diagnostics, KK.) as the reaction apparatus by means of 10 minutes of treatment at 95°C followed by repetition of 40 cycles with each cycle consisting of 15 seconds at 95°C, 5 seconds at 50°C and 20 seconds at 72°C.

Upon completion of the PCR the temperature was raised to 95°C, and after immediate cooling to 40°C, the temperature was held for 15 seconds and the temperature was raised again to 95°C at a rate of 20°C/sec. During heating, the fluorescent intensity at 640 nm and 710 nm was measured at every 0.2°C, the negative of the first derivative of the value (-dF/dT) was plotted, and the resulting peak was used to determine the melting temperature.

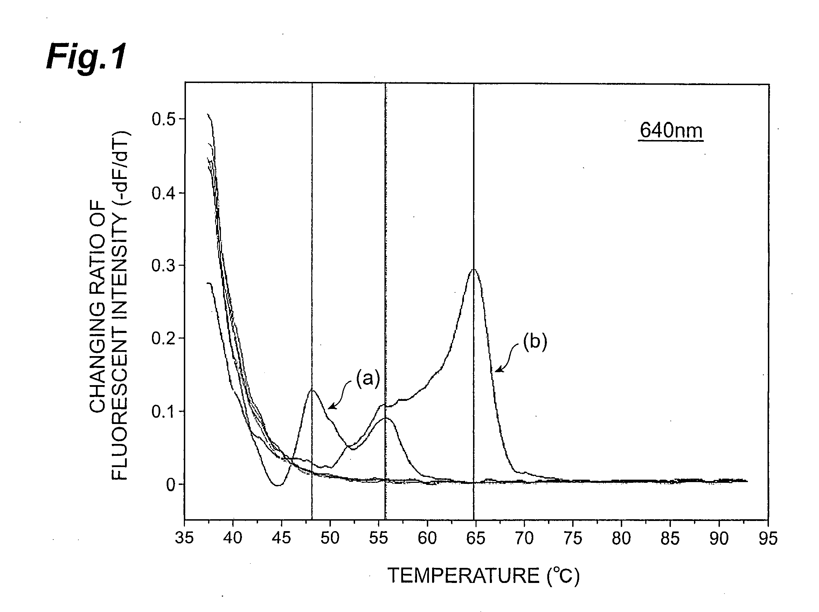

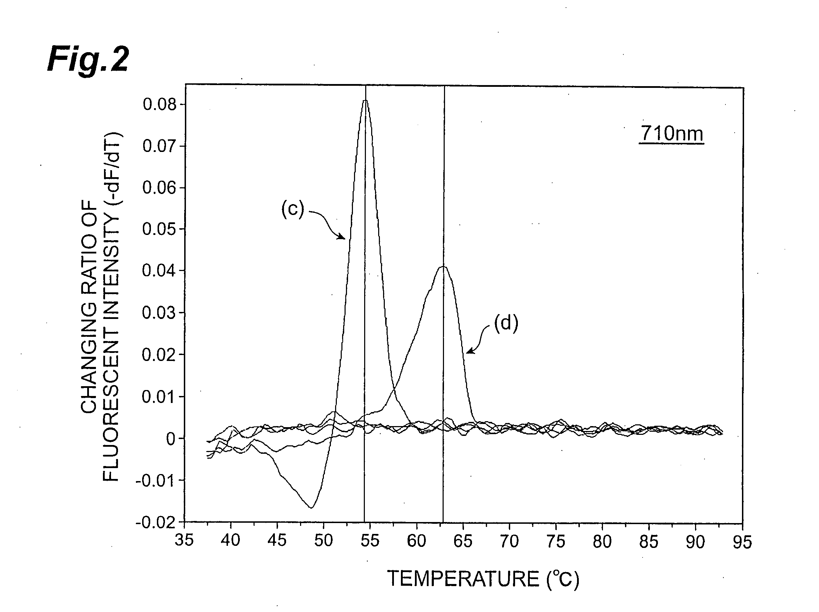

As a result, peaks were obtained representing melting temperatures of about 65°C at 640 nm for *M cerevisiae* SBC8034, of about 48°C and about 56°C at 640 nm for *Megasphaera cerevisiae* JCM6129, of about 63°C at 710 nm for *Pectinatus frisingensis* VTT-E-79100 and of about 54°C at 710 nm for *P. cerevisiiphilus* VTT-E-79105. Figs. 1 and 2 show melting curves representing the relationship between changing ratio of fluorescent intensity (-dF/dT) and temperature (°C). Fig. 1 shows melting curves for (a) *Megasphaera cerevisiae* JCM6129 and (b) *M cerevisiae* SBC8034 at 640 nm, and Fig. 2 shows melting curves for (c) *P. cerevisiiphilus* VTT-E-79105 and (d) *Pectinatus frisingensis* VTT-E-79100 at 710 nm. Strains other than these four strains did not exhibit peaks in the melting curve. The strains used for this example were the same as shown in Table 3.

Thus, it was confirmed that using the aforementioned primers and probes simultaneously allows multiple obligatory anaerobic gram-negative bacteria to be detected and distinguished.

### Industrial Applicability

The present invention allows different beer-spoilage microorganisms to be detected and distinguished simultaneously, and can therefore be used for quality control of beer.

## Claims

1. A polynucleotide comprising all or a portion of the nucleotide sequence as set forth in SEQ ID NO: 1.

2. A primer set for detecting *Malephilus cerevisiae* consisting of an oligonucleotide comprising the nucleotide sequence as set forth in SEQ ID NO: 2 and an oligonucleotide comprising the nucleotide sequence as set forth in SEQ ID NO: 3.

3. A primer set for detecting and distinguishing beer-spoilage microorganisms, consisting of an oligonucleotide comprising the nucleotide sequence as set forth in SEQ ID NO: 8, an oligonucleotide comprising the nucleotide sequence as set forth in SEQ ID NO: 3 and an oligonucleotide comprising the nucleotide sequence as set forth in SEQ ID NO: 6.

4. A probe set for detecting and distinguishing beer-spoilage microorganisms, consisting of an oligonucleotide comprising the nucleotide sequence as set forth in SEQ ID NO: 4, an oligonucleotide comprising the nucleotide sequence as set forth in SEQ ID NO: 7 and an oligonucleotide comprising the nucleotide sequence as set forth in SEQ ID NO: 5.

5. A kit for detecting and distinguishing beer-spoilage microorganisms, comprising a primer set according to claim 3 and a probe set according to claim 4.

6. A method for detecting *Malephilus cerevisiae* by gene amplification comprising a step of amplifying a nucleic acid fragment using a primer set according to claim 2 and a step of detecting the obtained nucleic acid fragment.

7. A method for detecting and distinguishing beer-spoilage microorganisms comprising a step of amplifying a nucleic acid fragment using a primer set according to claim 3 and a step of measuring the melting temperature of hybrids between the obtained nucleic acid fragment and a probe set according to claim 4.
